# EUROPEAN PATENT APPLICATION

(11) **EP 1 111 068 A1**
(43) Date of publication of application: **27.06.2001**
(21) Application number: 99125484.8
(22) Date of filing: 21.12.1999
(51) Int. Cl.: C12Q 1/68, C07H 21/00

(54) **Branched compound for use in nucleic acid detection and analysis reactions**

(71) Applicant: LION Bioscience AG, 69120 Heidelberg (DE); VBC Genomics GmbH, 1091 Wien (AT)
(72) Inventor: Schmidt, Wolfgang, Dr., 1030 Wien (AT); Hiller, Reinhard, Mag., 1120 Wien (AT); Huber, Martin, Mag., 1030 Wien (AT); Müller, Manfred, Dr., 3400 Klosterneuburg (AT)

(57) **Abstract**

The present invention concerns a branched compound for use in nucleic acid detection and analysis reactions, as well as a process for the synthesis thereof. The invention also concerns a process for a nucleic acid synthesis reaction making use of the branched compound as well as uses of the novel compound. The invention in addition concerns a kit comprising the branched compound.

## Description

### Background of the Invention

Genetic analysis often involves analysis of the nucleic acid sequence, structure or composition of a given organism or sample. frequently such analysis incorporates the step of, or requires nucleic acid amplification. One of the well known methods for nucleic acid amplification is the PCR, or polymerase chain reaction method also disclosed in US 4,683,195 and US 4,683,202. Here, a nucleic acid sample serves as a template for a polymerase dependant *in-vitro* replication starting from for two separate primers. Polymerases are enzymes capable of synthesizing RNA or DNA making use of RNA or DNA as a template. Often times the analysis is performed on RNA (ribonucleic acid), here amplification additionally requires an enzymatic reverse transcription into DNA (deoxyribonucleic acid), but equally often on DNA. PCR is becoming powerful tool in diagnostics. PCR kits are becoming available for the detection and analysis of various pathogenic organisms as well *e.g*. mutant alleles of human genes.

PCR is mostly performed *in-vitro, i.e.* in a tube whereby the components are mostly supplied in liquid format. Alternatively, one or more of the components, these usually being, a polymerase, a buffer, a template, two or more oligonucleotides, may be bound to some form of a solid-phase.

One very common problem with the PCR being performed in a standard non solid-phase format is the limitation with respect to the number of primer pairs that may be used simultaneously in one reaction. In contrast a solid-phase set-up would theoretically enable the use of tens to thousands of primer pairs.

More recently, it has been proposed to use one primer bound to a solid-support together with a free primer in solution in order to simultaneously amplify and bind a PCR product onto a surface (Oroskar, A. A., Rasmussen, S. E., et al., Detection of immobilized amplicons by EIISA-like techniques, Clinical Chemistry 42:1547 (1997)). The drawback of this approach is that the actual multiplexing , *i.e*. the use of a multitude of primer pairs in one reaction is not really facilitated as the free primers in solution may still spuriously anneal.

A solid-phase method is disclosed in US 5,641,658 or WO 96/04404 here, the oligonucleotides used in the reaction are bound to a support. Such a set-up would have a number of advantages over the standard PCR process, *e.g.* it would be easy to combine a multitude of primer pairs in one reaction without the drawbacks of false amplification products from false primer pairings. Thus, multiplexing would be facilitated.

A problem with such an approach is the efficiency of the reaction, thus also the product yield during amplification is poor and consequently the reliability of the entire process. Often these problems are associated with the fact that the reaction conditions *i.e*. the availability of the primer in the reaction are not optimal. One may envision that the 3' OH ends of the oligonucleotides are not available due to the fact that parts of the oligonucleotide are bound to the support. Such immobilized single-stranded DNAs which have conventionally been used are prepared by binding a single-stranded DNA at the terminal molecule or a suitable functional group introduced into the molecule. However, such conventional method has a drawback in that it is impossible to bind the single-stranded DNA to the carrier only at its terminal molecule by the use of the conventional method because an existing amino or hydroxy group, or other functional group artificially introduced, on the nucleotide molecules other than terminal also participates in the binding with the carrier. The immobilized DNA obtained with conventional methods is one in which the DNA molecules are bonded to the support at various sites of the strand. As will be recognized by those skilled in the art, such a molecule is poorly suited for providing sufficient experimental results in any aspects.

A further problem is that the molecules bound to the support are sterically hindered by the support itself from taking part in *e.g*. enzymatic reactions. EP0787205 discloses the use of linker between the oligonucleotide and the solid-support. However, the primary problem is not addressed here. The primers on a solid-support are not freely available in the reaction. Thus primers remain a limiting factor.

For efficient amplification to occur it would be desired to provide equimolar amount of each primer of a given pair of primers. Due to the fact that primers are bound to a support where an unequal distribution and binding of primers will occur stochastically it is impossible to achieve equimolar amounts of primer in the reaction.

Often reactive groups are used to bind the Primers to the support. Such reactive groups have been *e.g.* amino groups. It is known in the art that such groups are very unstable, consequently when a given primer pair is arrayed on a support wherein the terminal group of the primers are *e.g*. amino groups it is to be expected, based on the instability of the groups, that after arraying the primers in each pair will not be present equimolar manner.

One method for the covalent attachment of oligonucleotides on glass supports is to treat the glass with an aminosilane and couple a 5' amino-modified oligonucleotide via covalent bond formation using PDC (Guo et al. 1994 NAR 22:5456-5465). This procedure has two major technical drawbacks. As outlined above, the terminal primary amino group is unstable and therefore also not useful for long-term storage which is one pre-requisite in DNA chip technology. One should mention here that glass slides are often chosen as solid support in molecular DNA analysis and often referred to as DNA chips. Second, the generation of an aminoreactive glass support requires complete inactivation of unreacted groups after spotting in order to avoid a high background which in fact makes multiple solid-phase approaches entirely impossible.

One attempt to solve the problem of poor efficiency, poor yields, poor reproducibility could potentially be to incubate the primers with the template for extended amounts of time to enable improved binding efficiency however, it has been found that this has, depending on the solid support, severe drawbacks with respect to the enzymatic function. Some preferred solid supports such as glass will bind the enzyme, even denature it, and thus disable the reaction. It will be recognized by those skilled in the art that it is not desired to establish conditions in which the enzymatic function is decreased.

It was therefore an object of the present invention to provide for a process, means and substances that lead to a high efficiency in *in-vitro* solid-phase nucleic acid synthesis reactions, thus also to a higher product yield and consequently a higher reliability of such reactions.

It was further an object of the present invention to provide for a process, means and substances to be used in *in-vitro* solid-phase nucleic acid synthesis reactions that are suited for providing good experimental results in many aspects such as, equimolarity of oligonucleotide amount, reduction of background signal, reduction of falsely synthesized products, availability of oligonucleotides in the reaction.

It was a further object within the concept of the invention to provide for novel molecules capable of solving the above problems. Thus it was e.g. an object of the present invention to provide for molecules that are readily available in a DNA synthesis reaction and not sterically hindered by *e.g*. a solid-support or the like.

It was a also an object within the concept of the invention to provide for processes for making the molecules according to the invention.

It was a further object within the concept of the invention to provide for a kit comprising one or more of the molecules according to the invention.

Equally it was an object of the present invention to provide for a kit comprising compounds necessary for performing the process according to the invention.

Further objects of the invention are apparent to the skilled person from the specification.

The objects of the present invention were accomplished by providing for novel molecules with advantageous characteristics, processes for making these molecules, novel processes for in vitro nucleic acid synthesis for use with and without the novel molecules as well as kits containing molecules according to the invention for use in processes according to the invention.

Thus the object of the present invention was accomplished by providing for a branched compound for use in nucleic acid detection and analysis reactions, as represented in formula 1,

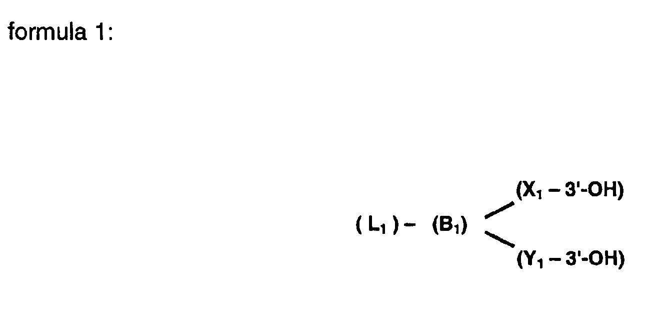

Here, **L**_{**1**} represents a linker moiety of variable length for attachment to a solid support, wherein **B**_{**1**} represents at least one branching moiety, **X**_{**1**} is one of at least two nucleic acid moieties, **Y**_{**1**} represents the second of at least two nucleic acid moieties and the nucleic acid moieties may be extended by a polymerase.

Herein, a solid-support may be any support used to perform solid phase reactions. solid phase reactions and solid-support reactions are used with equal meaning and shall be understood as such reactions in which one or more compounds is attached to a solid matter of any given shape or chemical structure. Thus a solid-phase reaction is a reaction in which at least one compound may not move freely through diffusion in the reaction.

The inventors observe a large enhancement in reaction efficiency when applying the compound according to the invention to e.g. *in-vitro* nucleic acid detection and analysis reactions.

The inventors attribute this unexpected behavior to the fact that two or more two nucleic acid moieties are in close spatial proximity.

The molecule according to the invention thus solves one of the problems in the prior art namely the availability of oligonucleotides for extension. Since the reaction product of *e.g.* X₁ may, in a subsequent cycle of a number of cycles of an in an *in-vitro* nucleic acid synthesis reaction, serve as a template for a reaction with Y₁ and since these are in close molecular vicinity, this reaction will run very efficiently. Also, the variable linker moiety **L**_{**1**}, makes it possible to overcome the drawbacks of known solid-phase reactions where the nucleic acids were in close vicinity to the solid-phase and thus cross-react with it to remain refrained from taking part in the actual reaction.

The compound according to the invention thus combines for the very first time a number of characteristics in itself which the inventors have found to be important for success in e.g. *in-vitro* nucleic acid detection and analysis reactios. (i) The compound according to the invention provides for the possibility to define the distance from a given solid-support. This is accomplished through the linker moiety L₁ which also serves to couple the compound to the solid-support. (ii) The compound according to the invention provides for the possibility to bring in close vicinity two or more nucleic acids (e.g. X₁ and Y₁) involved in one reaction, thus solving the problem of a lack of diffusion of *e.g.* nucleic acids in *in-vitro* nucleic acid synthesis reactions. (iii) The compound according to the invention provides for nucleic acids Xi and Y₁ which are not sterically hindered as the branching moiety B₁ permits them to become available in the reaction.

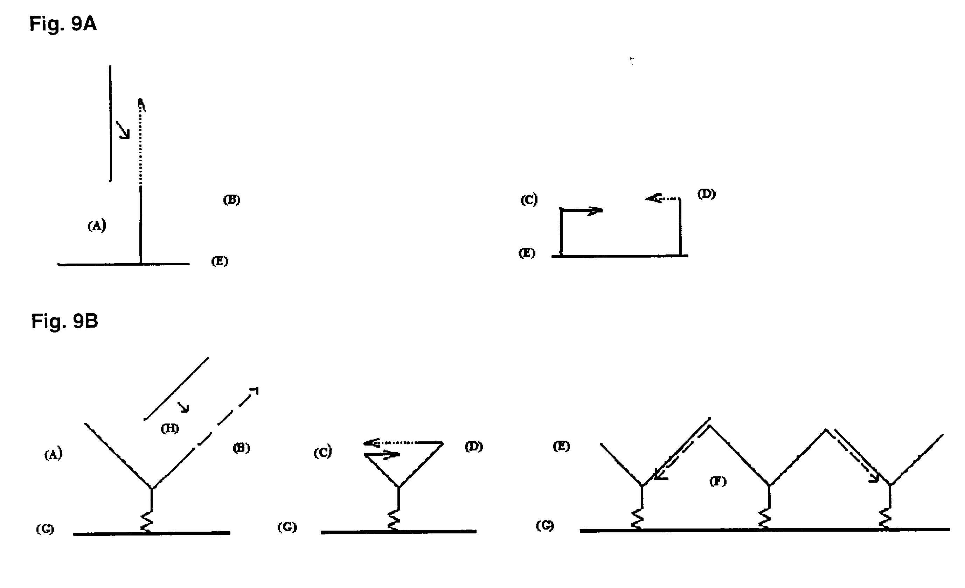

Figure 9B shall illustrate schematically some of the advantageous the branched compound according to the invention provides for.

In solid-phase, which is one way the molecule according to the invention may be used an efficient *in-vitro* nucleic acid synthesis reaction is dependant on either one of two events occurring, (i) a template molecule, whereby this stems from the sample with which the reaction was started, to reach a primer X₁, through diffusion or, (ii) a reaction product from a previous extension *e.g.* from X₁ to be in spatial vicinity and thus serve as a template for an extension by Y₁ (See also Fig. 9A and 9B).

It is obvious that the molecule according to the invention may comprise further branching moieties B₁ attached to one linker L₁ as well as more than 2 nucleic acids per branching moiety B₁. Herein, a branching molecule is defined as a molecule capable of binding 2 or more nucleic acids as well as a linker L₁.

For a better understanding we shall define the components of nucleic acid molecules as follows:

Deoxyribonucleic acid and ribonucleic acid are thread-like macromolecules, DNA comprising a chain of deoxyribnucleotides, and RNA comprising a chain of ribonucleotides. A "nucleotide" consists of a nucleoside and one or more phosphate groups; a "nucleoside" consists of a nitrogenous base linked to a pentose sugar. Typically, the phosphate group is attached to the fifth carbon ("5") hydroxyl group ("OH") of the pentose sugar; however, it can also be attached to the third-carbon hydroxy group ("3'-OH"). In a molecule of DNA, the pentose sugar is deoxy-ribose, in RNA, the pentose-sugar is ribose. The DNA bases are adenine ("A"), cytosine ("C"), guanine ("G"), and thymine ("T"). These base are the same for RNA except that uracil ("U") replaces thymine in natural RNA, i.e. RNA isolated from a living organism. Accordingly, the major nucleosides of DNA, collectively referred to as "deoxynucleosides", are as follows: deoxyadenosine ("dA"), deoxycytosine ("dC"), deoxyguanosine ("dG"), and deoxythymidine ("dT").

Herein, the abbreviations A, C, G and T shall refer to the DNA as well as RNA bases unless specified otherwise.

In this context nucleic acid moieties X₁ and Y₁, may be selected from the group comprising deoxynucleotides, dideoxynucleotides, ribonucleotides or modified nucleotides as specified above. If the term nucleotides is used, any of the above may be meant unless specified otherwise.

The inventors have found that it may, in some cases be advantageous to extend the distance between the branching molecule B and the oligonucleotides or oligonucleotide analogs by additionally adding one or more spacers to the molecule according to the invention. Here, the molecule additionally comprises spacers **SP**_{**1**} and **SP**_{**2**} with which the molecular distance between the free 3' OH ends of oligonucleotides or oligonucleotide analogs and the branch **B**_{**1**} may be defined. A possible structure is shown below in formula 2. Thus in a preferred embodiment of the present invention the branched compound additionally comprises two or more spacers **SP**_{**1**} and **SP**_{**2**} according to the following formula 2:

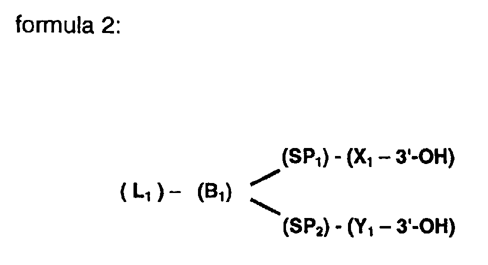

wherein **L**_{**1**} represents a linker moiety of variable length for attachment to a solid support, wherein **B**_{**1**} represents at least one branching moiety, wherein **X**_{**1**} is one of at least two nucleic acid moieties, wherein **Y**_{**1**} represent the second of at least two nucleic acid moieties, wherein the nucleic acid moieties may be extended by a polymerase and wherein **SP**_{**1**} and **SP**_{**2**} respectively represent spacer moieties which serve to define the distance between the branch moiety **B**_{**1**} and the nucleic acid moieties **X**_{**1**} **and Y**_{**1**}. The Inventors have found that these spacers enhance the reaction efficiency.

It is obvious that the spacers are optional and one may envision molecules where only one of the two oligonucleotides or oligonucleotide analogs are separated by a spacer from the branching molecule **B**_{**1**}**.** However, this is one of the essential features of the present invention as the variation of spacer length allows for adjustment to different template lengths and types.

Such spacers **SP**_{**1**} and **SP**_{**2**} are polymeric units comprising monomers. Although they may be chosen from the group comprising nucleotides, nucleotide analogs, abasic deoxyribose moieties, abasic ribose moieties, carbon chains which in turn may aliphatic or aromatic or even polyglycoles, they are not limited to these.

Also other molecules than those mentioned here may be used as a spacer as long as they fulfill the functional requirement of defining a distance between the branch and the free 3'-OH and do not hinder the polymerase from extending oligonucleotides or oligonucleotide analog. The inventors have empirically found that a suitable distance between the 3'-OH group and the branch molecule lies between 3 and 350 angstrom, preferably between 30 and 120 angstrom. These values were established by testing different spacer types and calculating the resulting lengths from the molecule type.

In a preferred embodiment **SP**_{**1**} and **SP**_{**2**} are polymeric units comprising monomers chosen from the group comprising nucleotides, nucleotide analogs, abasic deoxyribose moieties, abasic ribose moieties, carbon chains.

In an even more preferred embodiment of the invention the **SP**_{**1**} and **SP**_{**2**} are polymeric units comprising monomers of nucleotides or nucleotide analogs. Herein, nucleotides are to be understood as stretches of the same nucleotide or any given mixture of different nucleotides. The inventors have found between one and a hundred, more preferably between 10 and 30 to be suited, according to the distances described in more detail below.

The inventors have empirically found that a suitable distance between the 3'-OH group and the branch molecule lies between 3 and 350 angstrom, preferably between 30 and 120 angstrom. These values were established by testing different spacer types and calculating the resulting lengths from the molecule type.

In a preferred embodiment of the invention the branched compound further comprising a reactive group **(R**_{**1**}**)** as represented by formula 3. 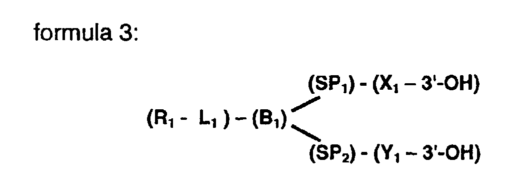 wherein **L**_{**1**} represents a linker moiety of variable length for attachment to a solid support, wherein **B**_{**1**} represents at least one branching moiety, wherein **X**_{**1**} is one of at least two nucleic moieties, wherein **Y**_{**1**} represent the second of at least two nucleic acid moieties, wherein the nucleic acid moieties may be extended by a Polymerase, wherein **SP**_{**1**} and **SP**_{**2**} respectively represent spacer moieties which serve to define the distance between the branch moiety **B**_{**1**} and the nucleic acid moieties **X**_{**1**} and **Y**_{**1**}

The inventors have found such a a reactive group R₁ to greatly facilitate the binding of the branched compound according to the invention to a given solid-support. Such a reactive group may be selected from a group comprising amino group, hydroxyl group, carboxyl group, aldehyde group, thiol group, organo-silane group and phosphate group.

Other groups, not mentioned here, accomplishing the purpose of binding the molecule according to the invention to a solid-phase are equally suited and shall equally be considered to be covered by the invention.

In a preferred embodiment of the branched compound according to the invention characterized in that the linker moiety **(L**_{**1**}**)** of variable length consists of polymeric units selected from the group comprising, nucleotides, nucleotide analogs, abasic deoxyribose moieties, abasic ribose moieties, carbon chains between about 3 carbons and 22 carbons, preferably between 3 carbons and 18 carbons in length, such chains may be aliphatic or aromatic, and polyglycoles.

Also other molecules than those mentioned here may be used as a linker moiety **(L**_{**1**}**)** of variable length as long as they fulfill the functional requirement of defining a distance between the solid-support and the branch moiety. The inventors have empirically found that a suitable distance between the branch moiety and the solid-support lies between 3 and 350 angstrom, preferably between 30 and 120 angstrom. These values were established by testing different linker moieties and calculating the resulting lengths from the molecule type.

In an even more preferred embodiment of the invention the linker moiety **(L**_{**1**}**)** of variable length consists of polymeric units comprising monomers of nucleotides or nucleotide analogs. Herein, nucleotides are to be understood as stretches of the same nucleotide or any given mixture of different nucleotides. The inventors have found between one and a hundred, more preferably between 10 and 30 to be suited, according to the distances described in more detail above.

As described above, two or more nucleic acid moieties (**X**_{**1**}**, Y**_{**1**}**, Z**_{**1**}, etc.) are coupled to the branch moiety **B**_{**1**}. It is an essential feature of the invention that at least to of these nucleic acid moieties carry a free 3' hydroxyl group and may thus be extended by a polymerase.

In a preferred embodiment of the invention X₁ and Y₁ consist of between 2 and 100 nucleotides in a more preferred embodiment of the invention between 10 and 30 nucleotides. Herein, oligonucleotide moiety will be used when the nucleic acid moieties consist of between 2 and 100 nucleotides.

Although it is possible that the two or more oligonucleotide moieties **X**_{**1**} and **Y**_{**1**} have the same sequence it is preferred if consist of different nucleotide sequences.

In a preferred embodiment the oligonucleotide moiety is characterized in that **X**_{**1**} and **Y**_{**1**} stem from the sense and anti-sense strand of a nucleic acid molecule. That means, if the were to anneal to a mixture of e.g. DNA, they would anneal to the plus and the minus strand respectively of a given DNA double helix.

In an even more preferred embodiment, the oligonucleotide moieties thus point towards each other with their free 3'-OH groups. Here, their positioning upon hybridization to a DNA double helix is so, that, a stretch of target DNA is located between the two positions of the respective oligonucleotide moieties X and Y.

The inventors have found that this embodiment leads to excellent results when the branched compound according to the invention is applied to solid-phase DNA synthesis reactions.

This embodiment is preferred but shall not limit the possibilities of designing the oligonucleotides which may in principle be designed in endless alternative ways of which the following are examples: The oligonucleotide moieties may (i) have the same sequence, (ii) have different sequences, (iii) have different sequences that hybridize to different targets, (iv) have different sequences that hybridize to the same target at different locations on the target, (v) have different sequences that hybridize to the plus and minus strand of one DNA double helix and many so on. One skilled in the art will readily recognize that there are numerous ways of designing the oligonucleotide moieties for numerous different purposes, all of which shall be covered by the scope of the invention.

As outlined above the reactive group R₁ serves to couple the branched compound to a solid-support. In a preferred embodiment the inventors have found it to be very advantageous if the reactive group is an organo-silane group. Formula four below depicts the entire molecule according to this preferred embodiment.

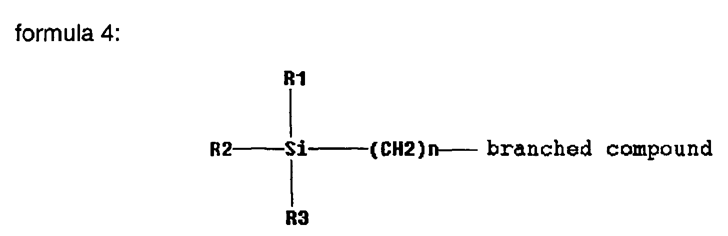

Here, R₁, R₂, and R₃ are identical or different alkoxy groups and "branched compound" refers to the entire compound according to the invention excluding the reactive group R1 and n is an integer from 0 to 18. This novel compound has surprisingly shown unexpected results when compared to similar molecules previously used in solid-phase enzymatic reactions with respect to but not limited to the following effects, its adsorption capacity to a solid-support, it's availability in enzymatic reactions, thus its contribution to the efficiency of *e.g.* solid-phase nucleic acid synthesis reactions.

The alkoxy groups R₁, R₂, and R₃ may, e.g. by methoxy, ethoxy or the like. Within the scope of the invention are organo-silane moieties comprising mixtures of different alkoxy groups. For example, R₁ may be a methoxy, R₂ an ethoxy and R₃ a methoxy. The skilled artisan is credited with the ability to discern alternative combinations which shall be within the scope of the invention.

On form of reactive group has proven to work particularly well here, **(R**_{**1**}**)** is a propyltrimethoxysilyl group according to the following formula 5,

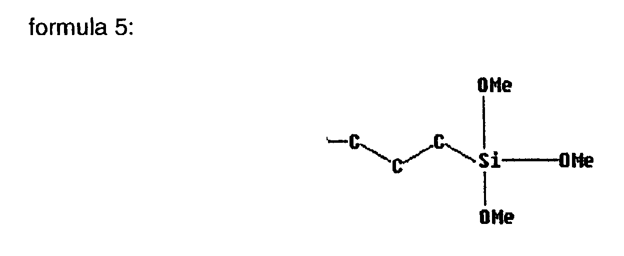

In a preferred embodiment of the present invention the branched compound according to the invention is attached to a solid-phase. Here, the branched compound is connected to a support via the linking moiety L₁ of which the reactive group R₁ has undergone a reaction with a group on the surface of the solid-support to form a covalent or non-covalent bond.

Such solid-phase may be chosen from the group comprising nitrocellulose, nylon, controlled-pore glass beads (CPG), polystyrene, activated dextran, modified polystyrene, styrene-acrylnitril-copolymers, polycarbonate, cellulose, polyamide and glass.

In a particularly preferred embodiment of the invention the solid-phase is glass. Such glass may be a glass slide, as used *e.g*. for microscopy, glass vessels or containers, glass fibers, glass beads or other Si comprising glass entities.

The compound according to the invention may be spotted onto, pipetted onto, sprayed onto or otherwise brought onto such a glass support. Possible methods are, application by means of a needle, capillary, dispenser and piezo pipette is preferable, *e.g*. an apparatus similar to the kind known for ink jet printers.

The compound according to the invention may be used in various ways some of which shall be mentioned here.

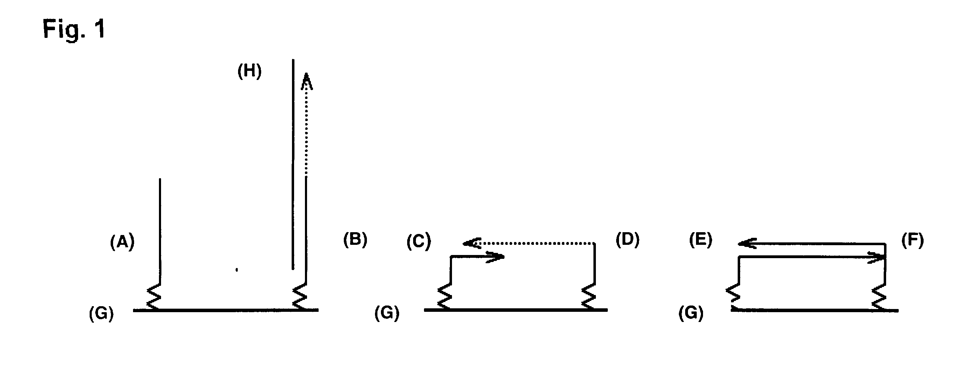

The compound is particularly suited and may thus be used in preferred embodiments of the invention for nucleic acid synthesis reactions, primer extension reactions, reverse transcription reactions of RNA into DNA or nucleic acid hybridization experiments (see also Figures 1 to 3).

In a preferred embodiment the compound is used in *in-vitro* nucleic acid synthesis reactions and hybridization experiments. Here, the compound may be bound to a solid support such as glass. The compound represents one or more nucleic acid probes to which a target, *i.e.* the sample is bound.

In a preferred embodiment it may be used in nucleic acid hybridization or synthesis reactions for identifying the sequence of a nucleic acid.

In a further preferred embodiment the branched compound may be used in nucleic acid hybridization or synthesis reactions for analyzing the expression pattern of genes. Here, oligonucleotides or nucleic acid fragments, *e.g*. PCR products represent the nucleic acid moieties according to the invention which are attached via the rest of the branched compound to a suited solid-support to form an array of various probes, labeled RNA or pre-amplified RNA is hybridized to the array and the positive hybridizations scored as expressed genes. Such methods are described in GB Pat. 2318791.

The compound according to the invention may be used to distinguish single base mismatches. The general principle of such an approach is disclosed in U.S. Pat. 5,700,638 where, such experiments are described in Example 2. US Pat. 5,552,270 also describes such an approach. Here, the compound according to the invention comprises an oligonucleotide of defined sequence. An array is generated comprising numerous different sequences each suited to test a defined sequence.

The branched compound according to the invention may be used to map genomes of organisms. Here, oligonucleotides or nucleic acid fragments, e.g. PCR products represent the nucleic acid moieties according to the invention which are attached via the rest of the branched compound to a suited solid-support to form an array of various probes, labeled genomic fragments are sequentially hybridized in numerous steps in such a way that the relationship between individual fragments becomes apparent. Such an approach is disclosed in U.S. Pat. 5,219,726.

One skilled in the art will find numerous further uses for the branched compound according to the invention all which shall equally be covered by the invention.

The invention shall also cover a process for a nucleic acid synthesis reaction of one or more selected regions of one or more target nucleic acids comprising the steps of a) combining the sample containing the target region with at least on nucleotide triphosphate, a thermally stable polymerase, a buffer and at least one branched compound according to the invention, b) exposing the reaction mixture to at least one temperature cycle including at least a high temperature denaturation phase and a lower temperature extension phase, and thereby producing at least a partially amplified product.

The inventors have found that the branched compound according to the invention gives unexpectedly good results when used on solid-phase in *in-vitro* DNA synthesis reactions (see also examples).

Thus in a preferred embodiment of the above process at least one branched compound according to the invention is bound to a solid-support.

In this process according to the invention it is preferred that the branched compound according to the invention comprises preferably between 3 and 100 nucleotides more preferably between 10 and 50 nucleotide most preferably between 12 and 25 nucleotides for each nucleic acid moiety. It is essential in this context that the nucleic acid moiety is of sufficient length to bind the desired target nucleic acid molecule with sufficient stringency. PNA-DNA hybrid oligonucleotides (see Finn, P. J. et al., N.A.R. 24, 3357-3363 (1996), Koch, T. et al., Tetrahedron Letters 36, 6933-6936 (1995), Stetsenko, D. A. et al., Tetrahedron letters 37, 3571-3574 (1996), Bergmann, F et al., Tetrahedron Letters 36 6823-6826 (1995) and Will, D. W. et al., Tetrahedron 51, 12069-12082 (1995)) are also considered as primers for the process of the present invention.

As a thermally stable polymerase, a DNA polymerase may be selected from the group comprising Taq DNA polymerase, Tth DNA polymerase or Klentaq (Taq DNA polymerase (-exo5'-3'), Korolev et al., (1995) Proc. Natl. Acad. Sci. USA 92, 9246-9268. The use of Taq DNA polymerase in the method of the present invention is especially preferred. One skilled in the art will recognize that numerous different thermally stable polymerases may be used.

Alternatively, as a thermally stable polymerase, a DNA polymerase which has a decreased discrimination against the four ddNTPs with respect to wild-type Taq DNA polymerase in the buffer or under the conditions used for thermal cycling is preferred. More preferably, a DNA polymerase Taq polymerase carrying a "Tabor-Richardson" mutation or a functional derivative thereof which also lacks 5'-3' exonuclease activity such as, for example, AmplitaqFS^{TM} (Taq DNA polymerase (-exo5'-3')(F667Y), Tabor and Richardson (1995), loc. cit.), Taquenase^{TM} (Taq DNA polymerase Δ235(-exo5'-3')(F667Y), Tabor and Richardson (1995), loc. cit.) and Thermosequenase^{TM} (Taq DNA polymerase (-exo5'-3')(F667Y), Tabor and Richardson (1995), loc. cit.) as well as mixtures thereof or other DNA polymerases and mixtures thereof which are thermally stable can be used in the process of the present invention. The use of Thermo Sequenase^{TM} or any other DNA polymerase having a high ability to incorperate ddNTPs in the method of the present invention is especially preferred.

Alternatively as a thermally stable polymerase, a DNA polymerase which has a decreased discrimination against labeled nucleotides may be used.

The present invention, *i.e.* the process also provides for the use of two or more polymerases in the process or additional enzymes such as amplification enhancing reagents such as thermally stable pyrophosphatase or enzymes which enhance the processivity of the polymerase such as PCNA (proliferating cell nuclear antigen) homologues. Enzyme mixtures may be equally applied.

The number of thermal cycles may range from about 1 to about 50 depending on the amount of template DNA and its purity. Generally, the inventors have found that very surprisingly extremely short cycles give good results. As the availability of the compound according to the invention is high in the process according to the invention the cycle period may be short, thus disadvantageous denaturing of proteins, e.g. the polymerase when in contact with glass occurs at a lower rate and the reaction may run efficiently without loss of function of enzyme.

Routinely, cycling consists of (i) a denaturing cycle, (ii) an annealing cycle and (iii) an extension cycle. Alternatively, only two cycles may be applied, (i) a denaturing cycle and (ii) an annealing and extension cycle.

Preferably the denaturing cycle is performed at between 100°C and 85°C, more preferably at between 98°C and 90°C, most preferably at between 96°C and 92°C. Preferably the annealing cycle is performed at between 80°C and 45°C, more preferably at between 70°C and 50°C, most preferably at between 60°C and 55°C.

Preferably the extension cycle is performed at between 80°C and 50°C, more preferably at between 75°C and 60°C, most preferably at between 73°C and 68°C.

Preferably the denaturing cycle is performed for 3 minutes, more preferably for 30 seconds, most preferably for under 10 seconds.

Preferably the annealing cycle is performed for 3 minutes, more preferably for 30 seconds, most preferably for under 10 seconds.

Preferably the extension cycle is performed for 3 minutes, more preferably for 30 seconds, most preferably for under 10 seconds, however the extension time vary depending on the length of the template, in particular the extension time may be raised if the template length increases.

Buffers components which can be used can include, but are not limited to, Tris-HCI at a pH of about 7.0 to 10 and concentration of about 2 to 60 mM, ammonium sulfate at a concentration of about 10-20 mM, preferably 15 mM, MgCl₂ at a concentration of about 1 to 10 mM, and optionally, about 0.05 mM mercaptoethanol, about 0.28% Tween® 20 and/or about 0.02% Nonidet® 40.

Nucleotide triphosphates are preferably deoxynucleotides and can be selected from, but are not limited to, dGTP, dATP, dTTP and dCTP. In addition, derivatives of deoxynucleotides, which are defined as those deoxynucleotides which are capable of being incorperated by a thermally stable DNA polymerase into nascent DNA molecules synthesized in the thermal cycling reaction, can also be used according to the invention. Such derivatives include, but are not limited to thionucleotides, 7-deaza-2'-dGTP, 7-deaza-2'-dATP as well as deoxyinosine triphosphate which may also be used as a replacement deoxynucleotide for dATP, dGTP, dTTP or dCTP. The above mentioned deoxynucleotides and derivatives thereof are preferably used at a concentration of about 50 µM to about 4 mM.

Preferably the nucleotides are mixes of all four nucleotides A, C, G, and T and at 200 µM each.

In a preferred embodiment one or more of the nucleotides incorporated are labeled. For example, single and differential labels may consist of the group comprising enzymes such as β-galactosidase, alkaline phosphatase and peroxidase, enzyme substrates, coenzymes, dyes, chromophores, fluorescent, chemiluminescent and bioluminescent labels such as FITC, Cy5, Cy5.5, Cy7, Texas-Red and IRD40(Chen et al. (1993), J. Chromatog. A 652: 355-360 and Kambara et al. (1992), Electrophoresis 13: 542-546), ligands or haptens such as biotin, and radioactive isotopes such as ³H, ³⁵S, ³²P ¹²⁵l and ¹⁴C.

In one embodiment of the method of the invention, the nucleic acid molecule to be amplified can be present in the form of total genomic DNA, which is preferably in an uncloned or unpurified form. Preferably, the genomic DNA has a length greater than or equal to 2 kb. Generally all forms of template may be used, *e.g*. purified nucleic acids, *i.e.* nucleic acids where one fraction may be enriched or not, one example being plasmid DNA the other purified genomic DNA. The process may be suited for use with complex mixtures of DNA such being purified but not substantially fractionated genomic DNA or non-complex mixtures such being purified and substantially fractionated DNA e.g. plasmid DNA.

In a further preferred embodiment of the method of the invention, the nucleic acid molecule to be amplified can be present in the form of RNA. One polymerase or a mixture of two polymerases maybe utilized: a first DNA polymerase for example, Taq polymerase, and a second DNA polymerase with the capability to reverse transcribe RNA into DNA preferably Taq DNA polymerase (Jones et al., Nucl. Acids Res. 17: 8387-8388 (1989)) or Tth DNA polymerase (Myers et al., Biochemistry 30: 7666-7672 (1991)).

While the foregoing has been set forth in detail, the Examples are presented for elucidation, and not limitation. Modifications and improvements on the compound and or process according to the invention disclosed above which are within the purview and abilities of those in the art are included within the scope of the claims.

The invention also covers a kit for use in molecular biology or chemistry comprising at least the compound according to the invention. The kit may also comprise other reagents or enzymes such as buffers, nucleotides or the like.

While the foregoing has been set forth in detail, the Examples are presented for elucidation, and not limitation. Modifications and improvements on the compound and or process according to the invention disclosed above which are within the purview and abilities of those in the art are included within the scope of the claims.

### Example 1:

DNA Oligonucleotides were synthesized using standard cyanoethyl posphoramidite chemistry on an ABI 392 DNA/RNA Synthesizer (Caruthers MH, Barone AD, Beaucage SL, Dodds DR, Fisher EF, McBride LJ, Matteucci M, Stabinsky Z, Tang JY Chemical synthesis of deoxyoligonucleotides by the phosphoramidite method, Methods Enzymol 154, 287-313 (1987)). Primary amino groups with an hexyl carbon atom spacer were introduced using the N-MMT-C6-aminomodifier (Cruachem Cat. No. 22-8401-17). Oligonucleotides were synthesized using the trityl-on mode and purified via HPLC (BioCad, PE Biosystems) using Oligo R3 reversed phase chromatography media (PE Biosystems Cat. No. 1-1339-06) and an on-column detritylation standard protocol. The oligonucleotides contained specific 20 or 19 nucleotides together with a T₉ or T₁₅ spacer and were designed for the amplification of a 223 bp fragment of the polylinker sequence of pBlueScript KS + (Stratagene Cat. No. 212207).

Primer sequences:

SEQ ID NO 1:

"BSfor": 5'-NH2-C6-TTTTTTTTTAAGCGCGCAATTAACCCTCA-3'-OH

SEQ ID NO 2:

"BSrev": 5'-NH2-C6-TTTTTTTTTTTTTTTAAAACGACGGCCAGTGAGC-3'-OH

The single branched amplification molecule was synthesized using cyanoethyl posphoramidite chemistry on an ABI 392 DNA/RNA Synthesizer. The 3'terminal amino group was introduced by starting synthesis with an aminolinker column (Cruachem Cat. No. 19-7850-80). The 5'-branch was introduced using either symmetric or assymmetric branching CE phosphoramidites (Cruachem Cat. No. 22-8424-17 or 22-8425-17). Reactive carboxyl groups at the 5'-ends were introduced using Carboxy-dT CE phosphoramidite (Glen Research Cat.No. 10-1035-02), while primary amino groups were introduced using the N-MMT-C6-aminomodifier (Cruachem Cat. No. 22-8401-17).

Nucleic acid sequences:
SEQ ID NO 3:
Branch1 (5'-NH2-C6-TTT)2 – Branch - TTTTTTTTTTTTTTT-3'-NH2
SEQ ID NO 4:
Branch2 (5'-dT-COOH)2 – Branch - TTTTTTTTTTTTTTT-3'-NH2

The aminomodified branch (2 nmol) was incubated with the aminomodified oligonucleotides in 0.2 % (w/v) 1,4-phenylene-diisothiocyanate (PITC) in pyrrimidine/dimethylformamide 1/9 in a 100 µl volume at ambient temperature for 2 hours or longer. Purification was then achieved by precipitation with 0.3 M sodium acetate / ethanole and electrophoretic separation on a 15 % polyacrylamide / 8 M urea gel. (Size fractionation: 21 nt + 29 nt + 34 nt = 84 nt)

The carboxymodified branch (2 nmol) was incubated with the aminomodified oligonucleotides in molar excess. The reaction was accomplished by incubation of the carboxy and amino components in 100 µl aqueous solution containing 9 mg of the peptide condensing agent 1-ethyl-3-(3-dimethylaminopropyl)-carbodiimide (EDC) 6 hours at 4 °C. Purification was then achieved by precipitation with 0.3 M sodium acetate / ethanol and electrophoretic separation on a 15 % polyacrylamide / 8 M urea gel. (Size fractionation: 17nt + 29nt + 34nt = 80nt)

Purified single branched amplification molecules containing the two covalently bound primers were attached via their 3'-amino group to an amino-reactive solid support *i.e.* silylated slides (Cell Associates, Cat. No. CSS-100). The single branched amplification molecules were resuspended in 100 mM sodium carbonate buffer at pH 9.0 at a concentration of 100 µM. During the arraying process using a GMS 417 Arrayer (Genetic MicroSystems) which was adjusted to 30 hits per dot *(i.e.* spot) up to 0.75 pmoles of oligonucleotide were immobilized in spots of 150 µm diameter on the glass support. After spotting slides were incubated for 4 hours in a humid chamber to allow rehydration of the DNA, washed once in 0.2% SDS, twice in water, and incubated for 5 minutes in 0.25 % sodium borohydride in phosphate buffered saline for blocking free aminoreactive groups of the glass slide. Arrays are then submerged in water 2 minutes at 95 °C, transferred to 0.2% SDS for 1 minute and rinsed twice in water. After drying, arrays can be stored in the dark at ambient temperature.

Amplification reactions were performed in a 25 µl volume containing 50 mM Tris-HCI at pH 8.5, 30 mM KCI, 3 mM magnesium chloride, 12.5 µg bovine serum albumine, 200µM each dGTP, dATP and dTTP, 100 µM dCTP, 4 µM Cy5-dCTP (Amersham Pharmacia Biotech, Cat. No. PA 55021), 0.1 unit Taq DNA Polymerase and 3 fmole of template DNA (pBlueScript KS + plasmid DNA). The reaction chamber was formed by sealing the reaction droplet on the slide with FrameSeal Slide Chambers (Biozym Diagnostik, Cat. No. 621741). The silde is then attached to a modified frame (originally designed for holding glass capillaries) which fits to the reaction chamber of the thermal-cycler device (Idaho Technology, Thermo-Cycler 1605). Reactions are cycled with the following profile one initial cycle at 94°C for 30 seconds, thirty cycles for 10 seconds at 94 °C, for 15 seconds at 60°C, for 25 seconds at 74 °C and one final cycle for 30 seconds at 74°C.

After the incubation in the thermal-cycler slides were removed and the frame seals lifted off. The slides were transferred to a micro-array washing device (Telechem International, Arraylt Microarray Wash Station) and rinsed with 0.2 % SDS for 15 minutes, cleaned in water for 15 minutes and dried at ambient temperature. Scanning of the fluorescent PCR products was accomplished in a fluorescent scanning device (Genetic Microsystems, Array Scanner).

### Example 2:

Oligonucleotides were synthesized using standard cyanoethyl posphoramidite chemistry on an ABI 392 DNA/RNA Synthesizer. Primary amino groups with an hexyl carbon atom spacer were introduced using the N-MMT-C6-aminomodifier (Cruachem Cat. No. 22-8401-17). Oligonucleotides were synthesized using the trityl-on mode and purified via HPLC (BioCad, PE Biosystems) using Oligo R3 reversed phase chromatography media (PE Biosystems Cat. No. 1-1339-06) and an on-column detritylation standard protocol.

The purified oligonucleotide or single branched amplification molecule containing the 5' hexylamino modification (100 pmoles) was incubated in 100 µl of a 100 mM 1,4-phenylene diisothiocyanate (DITC) containing solution of pyrridine : dimethylformamide (ratio 1 : 9) for 2 hours at ambient temperature

After standard precipitation with ethanol/sodium acetate the oligonucleotide was dissolved in 100 µl of a 1 % 3-aminopropyltrimethoxysilane solution in 95 % acetone/water and incubated for 2 hours at ambient temperature (Reaction B). After standard precipitation the oligonucleotide bearing the glass-reactive silane group was re-dissolved in 95 % aqueous acetone and stored at 4 °C. Attachment to various supports, such as glass was accomplished by spotting (Reaction C).

### Figure Captions:

### Fig.1:

Fig. 1 shows a schematic representation of a solid-phase *in-vitro* nucleic acid synthesis reaction. The figure shows a oligonucleotide wherein, the oligonucleotide as an arrow and an organo-silane moiety as zig-zag line, both coupled via the organo-silane moiety to a solid support (G). Here, an *in-vitro* nucleic acid synthesis reaction is schematically shown. A target molecule (H) anneals to the oligonucleotide (B) where it is used as a template and oligonucleotide is extended by a polymerase. Thereafter, the extended oligonucleotide may serve as a template for a further oligonucleotide (C) which must be in sufficiently close vicinity. The oligonucleotide (C) now uses the template (D) and is extended to the full-length product (E). As schematically shown here, efficient synthesis relies on equimolarity and close vicinity of two or more different oligonucleotide required in one reaction.

### Fig. 2:

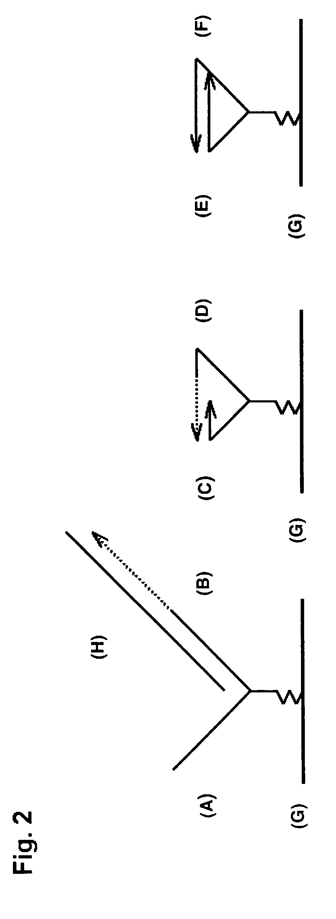

Fig. 2 shows a schematic representation of a solid-phase *in-vitro* nucleic acid synthesis reaction. The figure shows a branched compound according to the invention wherein, the branched compound comprises two free 3'-OH groups and an organo-silane moiety as zig-zag line, both coupled via the organo-silane moiety to a solid support (G). Here, an *in-vitro* nucleic acid synthesis reaction is schematically shown. A target molecule (H) anneals to the nucleic acid moiety of the branched compound (B) where it is used as a template and the nucleic acid moiety is extended by a polymerase. Thereafter, the extended nucleic acid moiety may serve as a template for a further nucleic acid moiety (C) which is by definition in sufficiently close vicinity. The nucleic acid moiety (C) now uses the template (D) and is extended to the full-length product (E).

### Fig. 3:

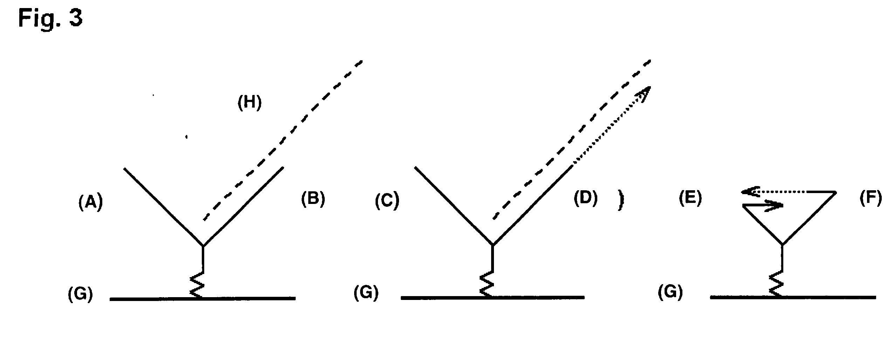

Fig. 3 shows a schematic representation of a solid-phase *in-vitro* nucleic acid synthesis reaction starting from mRNA including the step of prior reverse transcription coupled to an amplification step. The figure shows a branched compound according to the invention wherein, the branched compound comprises two free 3'-OH groups and an organo-silane moiety as zig-zag line, both coupled via the organo-silane moiety to a solid support (G). Here (A to F), an *in-vitro* nucleic acid reverse transcription with coupled amplification reaction is schematically shown. A target mRNA molecule (H) anneals to the nucleic acid moiety (B) where it is used as a template and the nucleic acid moiety is extended by a polymerase with reverse transcriptase activity. Thereafter, the extended nucleic acid moiety may serve as a template for a further nucleic acid moiety (C) which is by definition in sufficiently close vicinity. The nucleic acid moiety (C) now uses the template (D) and is extend to the full-length product (E). As schematically shown here, efficient synthesis relies on equimolarity and close vicinity of two or more different nucleic acid moiety required in one reaction. The compound according to the invention takes place in reverse transcription as well as subsequent amplification.

### Fig. 4:

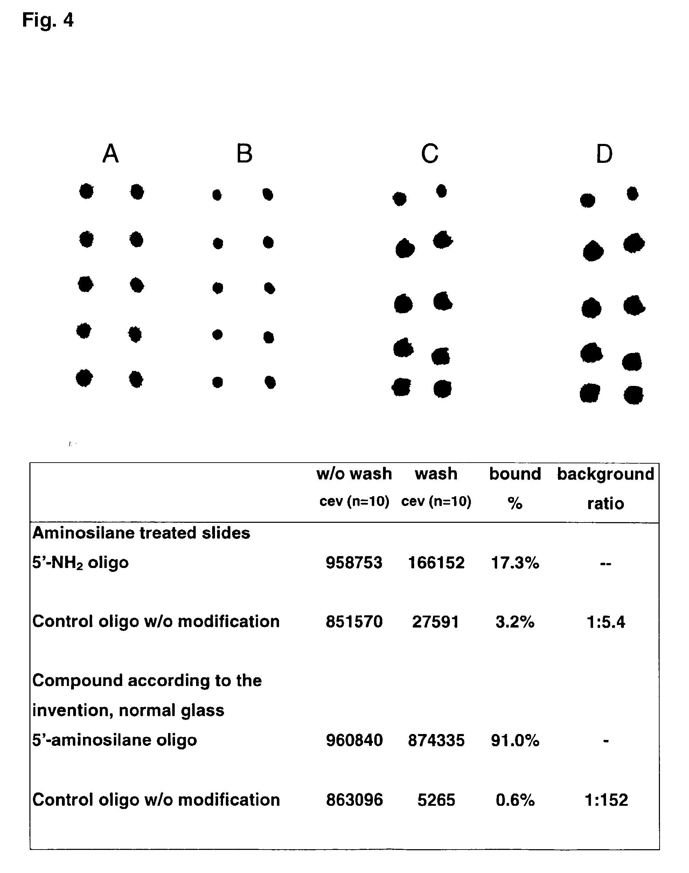

Fig. 4 shows a comparison between binding amino-modified oligonucleotides to silane-treated glass (panels A, B) and binding oligonucleotides carrying a terminal silane group to normal glass (panels C, D). In a preferred embodiment of this invention, the compound according to the invention carries such a organo-silane group as shown here in combination with a standard oligonucleotide. In a preferred embodiment of the invention, the reactive group R₁ is an organo-silane group and thus the compound according to the invention will spot as in this example. The 3' ³²P-labeled oligonucleotides (10.000 cpm) were spotted onto the respective glass slides and scanned (panels A, C). As a control also unmodified oligonucleotides were spotted onto both types of slides (not shown, see Table) to determine unspecific background binding. After extensive washing and rinsing steps the slides were dried and scanned again (panels B, D). Herein, cev (counts equivalent value) were determined by quantification of the Phosphor Imager gel scans using the ImageQuant software.

### Fig. 5:

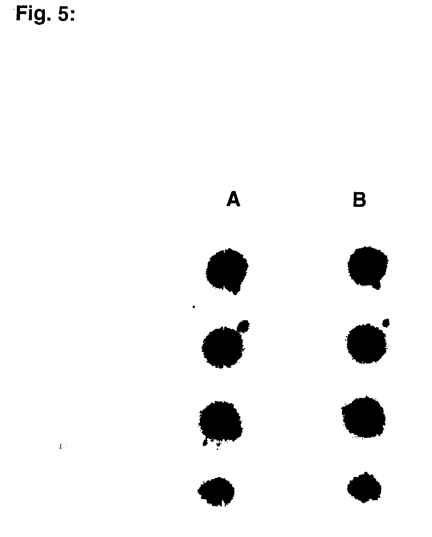

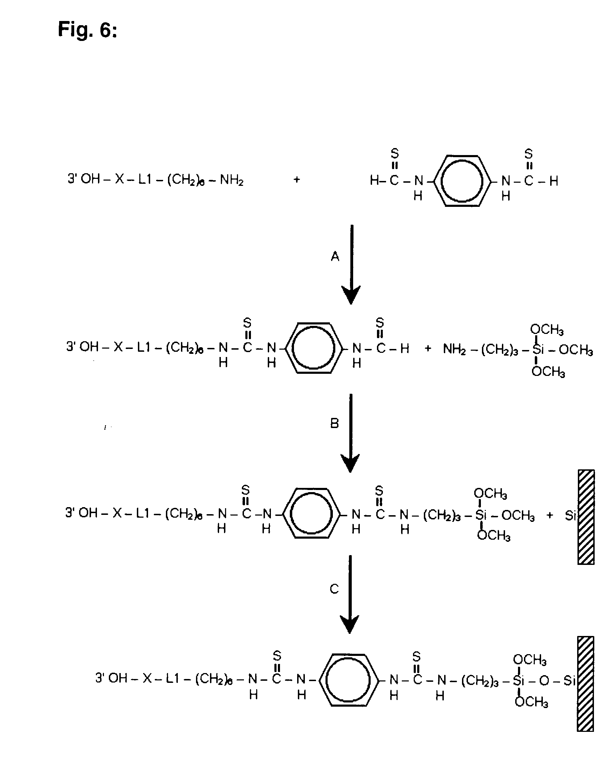

In a preferred embodiment of this invention, the branched compound according to the invention carries such a organo-silane group as shown here in combination with a standard oligonucleotide. In a preferred embodiment of the invention, the reactive group R₁ is an organo-silane group and thus the compound according to the invention will spot as in this example. Fig. 5 shows an experiment for the determination of the binding capacity of an organo-silane modified oligonucleotide to normal untreated glass. 1 nmol of an amino-modified oligonucleotide was spiked with 1 fmol of gel-purified 3'-end labeled oligonucleotide (10.000 cpm) and derivatized with an organo-silane according to the invention, spotted four times onto the slides and scanned (panel A). The slides were then washed, dried and scanned again (panel B). 98,7 % of the silane-treated oligonucleotides were covalently bound to the glass support.

### Fig. 6:

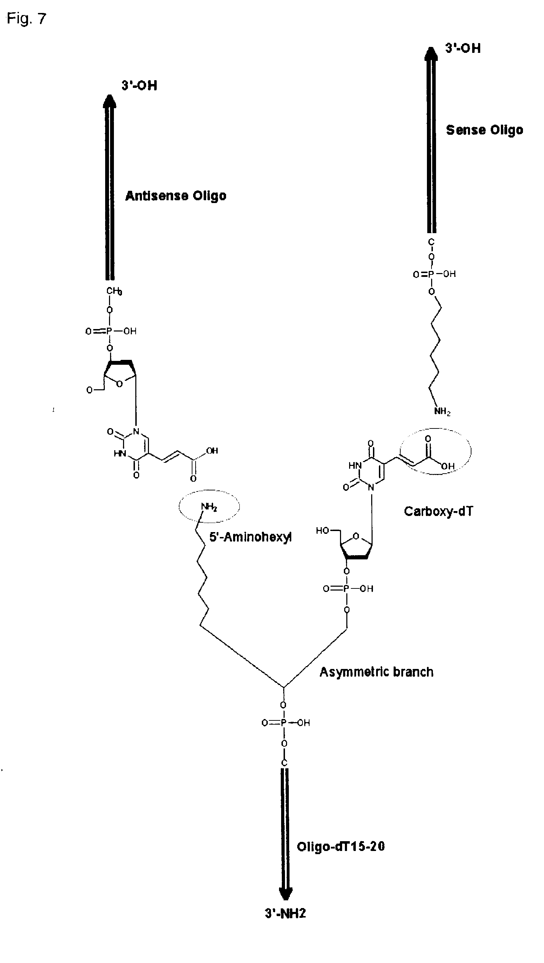

Fig. 7 shows schematically the reactions leading to the derivatization of an oligonucleotide with an organo-silane moiety according to a preferred embodiment of the invention. An oligonucleotide containing a 5' hexylamino modification is first reacted with 1,4-phenylene diisothiocyanate (reaction A) and further reacted with 1, 3-aminopropyltrimethoxy-silane (reaction B). The compound bearing the glass-reactive silane group can then be attached to *e.g.* glass supports (reaction C).

### Fig. 7:

Fig. 7 shows a preferred embodiment of the compound according to the invention. Here, the nucleic acid moieties were attached separately.

### Fig. 8:

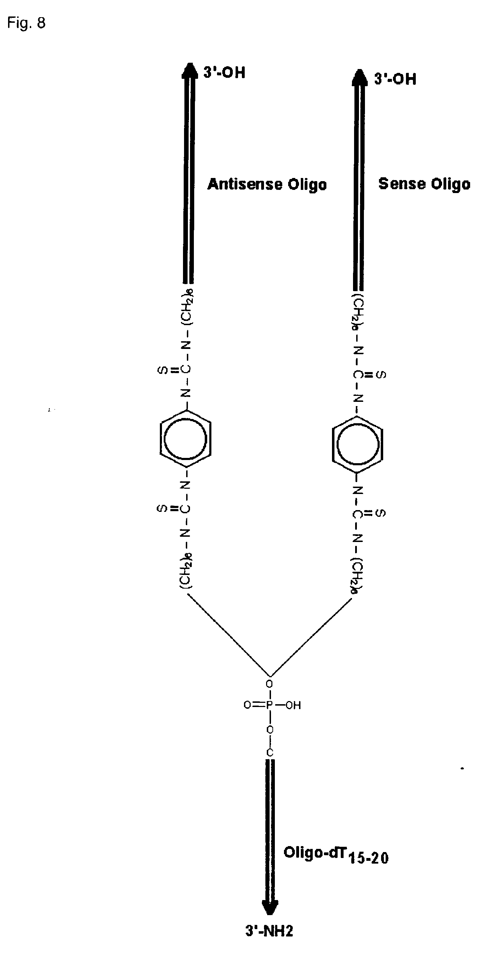

Fig. 8 shows a preferred embodiment of the compound according to the invention. Here, the nucleic acid moieties were attached separately using two identical bifunctional groups.

### Fig. 9 A:

Fig. 9 A shows a prior-art nucleic acid synthesis on solid-phase (E) . One must note, that template (A) is supplied through diffusion only. In the even that, the oligonucleotides of sense (C) and antisense (D) are not in sufficient vicinity or are sterically hindered (not shown here) they may not serve as template upon elongation. Thus, once the oligonucleotide (B) is elongated factors such as concentration, steric hindrance etc. will decide whether it may serve as a template in a subsequent cycle.

### Fig. 9 B:

Fig. 9 B shows a nucleic acid synthesis process on solid-phase (G) according to the invention. Once diffusion template (H) reaches any of the nucleic acid moieties, the may serve themselves as a template (C and D) or neighboring branched compounds (E and F) may serve as templates. Thus, the process according to the invention overcomes fundamental problems connected to the prior-art.

## Claims

1. Branched compound for use in nucleic acid detection and analysis reactions,
as represented in formula 1, 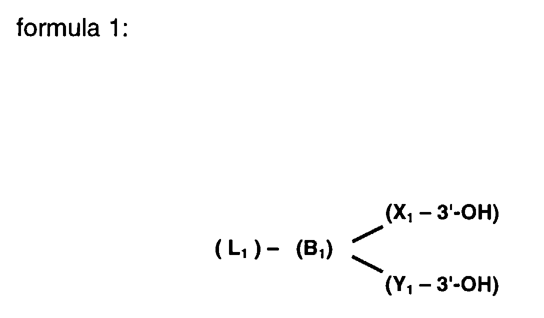 wherein **L**_{**1**} represents a linker moiety of variable length for attachment to a solid support,
wherein **B**_{**1**} represents at least one branching moiety,
wherein **X**_{**1**} is one of at least two nucleic acid moieties,
wherein **Y**_{**1**} represents the second of at least two nucleic acid moieties and
wherein the nucleic acid moieties may be extended by a polymerase.

2. Branched compound according to claim 1,
as represented in formula 2, 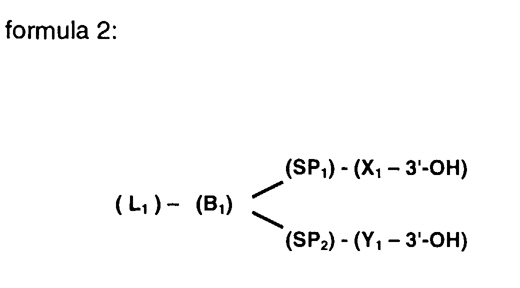 wherein **L**_{**1**} represents a linker moiety of variable length for attachment to a solid support,
wherein **B**_{**1**} represents at least one branching moiety,
wherein **X**_{**1**} is one of at least two nucleic acid moieties,
wherein **Y**_{**1**} represent the second of at least two nucleic acid moieties,
wherein the nucleic acid moieties may be extended by a polymerase and
wherein SP₁ and SP₂ respectively represent spacer moieties which serve to define the distance between the branch moiety B₁ and the nucleic acid moieties X₁ and Y₁

3. Branched compound according to claims 1 to 2,
further comprising a reactive group (**R**_{**1**}) as represented by formula 3, 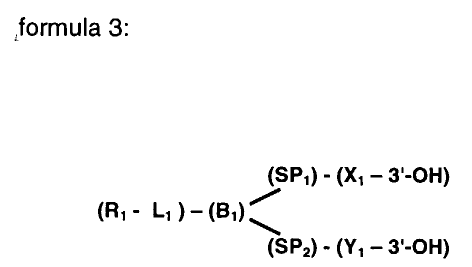 wherein **L**_{**1**} represents a linker moiety of variable length for attachment to a solid support,
wherein **B**_{**1**} represents at least one branching moiety,
wherein **X**_{**1**} is one of at least two nucleic moieties,
wherein **Y**_{**1**} represent the second of at least two nucleic acid moieties,
wherein the nucleic acid moieties may be extended by a Polymerase,
wherein **SP**_{**1**} and **SP**_{**2**} respectively represent spacer moieties which serve to
define the distance between the branch moiety **B**_{**1**} and the nucleic acid moieties **X**_{**1**} and **Y**_{**1**} and,
wherein R₁ is a reactive group selected from a group comprising amino group, hydroxyl group, carboxyl group, aldehyde group, thiol group, organo silane group and phosphate group

4. Branched compound according to claims 1 to 3,
characterized in that **SP**_{**1**} and **SP**_{**2**} are polymeric units comprising monomers chosen from the group comprising nucleotides, nucleotide analogs, abasic deoxyribose moieties, abasic ribose moieties, carbon chains and polyglycoles.

5. Branched compound according to claims 1 to 4,
characterized in that the linker moiety of variable length **(L**_{**1**}**)**consists of polymeric units selected from the group comprising, nucleotides, nucleotide analogs, abasic deoxyribose moieties, abasic ribose moieties, carbon chains and polyglycoles.

6. Branched compound according to claim 1 to 5,
characterized in that **X**_{**1**} **and Y**_{**1**} consist of between 2 and 100 nucleotides.

7. Branched compound according to claim 6,
characterized in that **X**_{**1**} **and Y**_{**1**} consist of between 10 and 30 nucleotides.

8. Branched compound according to claim 1 to 7,
characterized in that the nucleic acid moieties **X**_{**1**} and **Y**_{**1**} are oligonucleotide moieties and consist of different nucleotide sequences.

9. Branched compound according to claim 8,
characterized in that **X**_{**1**} and **Y**_{**1**} stem from the sense and anti-sense strand of a nucleic acid molecule.

10. Branched compound according to claims 1 to 9,
characterized in that **(R**_{**1**}**)** is a terminal organo-silane group and the entire compound is thus structured according to formula 4: 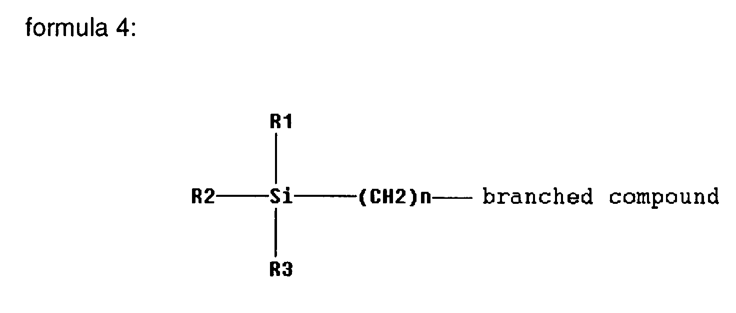 wherein, R₁, R₂, and R₃ are identical or different alkoxy groups wherein, the term "branched compound" is the compound according to the above claims excluding the reactive group R₁ and wherein,
n is an integer from 0 to 18.

11. Branched compound according to claims 1 to 10,
characterized in that **(R**_{**1**}**)** is a propyltrimethoxysilyl group according to the following formula 5,

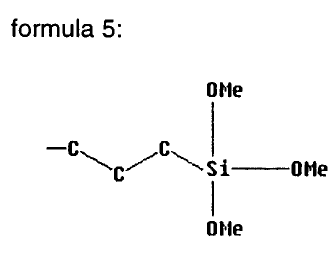

12. Branched compound according to claims 1 to 11,
characterized in that it is attached to a solid-phase with the reactive group **(R**_{**1**}**)**

13. Branched compound according to claim 12,
characterized in that the solid-phase is chosen from the group comprising nitrocellulose, nylon, controlled-pore glass beads (CPG), polystyrene, activated dextran, modified polystyrene, styrene-acrylnitril-copolymers, polycarbonate, cellulose, polyamide and glass.

14. Branched compound according to claim 13,
characterized in that the solid-phase is glass.

15. Use of a branched compound according to claims 1 to 14,
in nucleic acid synthesis reactions.

16. Use of a branched compound according to claims 1 to 14
in primer extension reactions.

17. Use of a branched compound according to claims 1 to 14,
in reverse transcription reactions of RNA into DNA.

18. Use of a branched compound according to claims 1 to 14,
in nucleic acid hybridization experiments.

19. Use of a branched compound according to claims 1 to 14,
in nucleic acid hybridization or synthesis reactions for identifying the sequence of a nucleic acid.

20. Use of a branched compound according to claims 1 to 14,
in nucleic acid hybridization or synthesis reactions for analyzing the expression pattern of genes.

21. Process for the synthesis of a branched compound according to claims 1 to 14
characterized in that,
a) in a first step a linker moiety L₁ is supplied or synthesized,
b) in a second step at least one branching moiety B₁ is reacted with or synthesized onto the linker reagent,
c) in a third step the first of at least two nucleic acid moieties X₁ is reacted with or synthesized onto the branching moiety B₁,
d) in a fourth step the second of at least two nucleic acid moieties Y₁ is reacted with or synthesized onto the branching moiety B₁,

22. Process for the synthesis of a branched compound according to claim 21,
characterized in that,
a) in a first step a first linker **L**_{**1**} moiety is synthesized online,
b) , whereby between 1 and 100 monomers are added with a terminal nucleotide carrying a OH-group,
c) an asymmetric branching moiety B₁ is bound to the linker through either a peptide bond or phosphodiester bond,
d) one of at least two protecting groups is released from the asymmetric branching moiety B₁,
e) an oligonucleotide or oligonucleotide **X**_{**1**} analog is synthesized in reverse direction (5' to 3'),
f) the nucleic acid moiety **X**_{**1**} from step d) is capped with a protecting group
g) a second of at least two protecting groups is released from the asymmetric branching moiety **B**_{**1**},
h) a further nucleic acid moiety **Y**_{**1**} is synthesized in reverse direction (5' to 3').

23. Process for the synthesis of a branched compound according to claim 21
characterized in that,
one or more of the nucleic acid moieties **X**_{**1**}**, Y**_{**1**}, are synthesized separately and bound to the branching moiety upon synthesis.

24. Process according to any of the claims 21 to 23,
characterized in that the branching moiety **(B**_{**1**}**)** is an asymmetric branching moiety with the following structure, 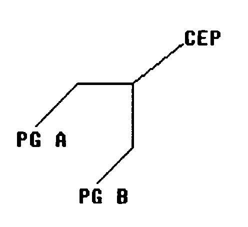 wherein PG A is a protecting group chosen from the group comprising LEV (levulinyl), DMTO (trityl), MMT (monomethoxytrityl),
wherein PG B is a protecting group chosen from the group comprising LEV (levulinyl), DMTO (trityl), MMT (monomethoxytrityl),
wherein CEP stands for cyanothylphosphoramidite and wherein PG A and PG B may not be identical.

25. Molecule according to any of the claims 21 to 23
characterized in that the branching moiety **(B**_{**1**}**)** is an asymmetric branch with the following structure, 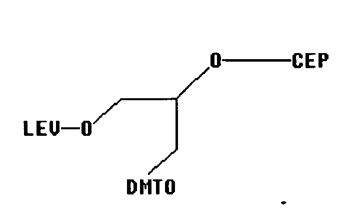 wherein LEV stands for the protecting group levulinyl,
wherein DMTO stands for the protecting group trityl and
wherein CEP stands for cyanoethylphosphoramidite.

26. Process for a nucleic acid synthesis reaction of one or more selected
regions of one or more target nucleic acids comprising the steps of
a) combining the sample containing the target region with at least on nucleotide triphosphate, a polymerase, a buffer and at least one branched compound according to claim 1-14,
b) exposing the reaction mixture to at least one temperature cycle including at least a high temperature denaturation phase and a lower temperature extension phase, and thereby producing at least a partially amplified product.

27. Process according to claim 26,
wherein the compound according to the invention is bound to a solid support.

28. Process according to claims 26 and 27,
characterized in that,
the buffer used contains between Bovine Serum Albumine (BSA) at a concentration of between 0.1 µg per µl and 5 µg per µl.

29. Process according to claim 28,
characterized in that,
the buffer used contains Bovine Serum Albumine (BSA) at a concentration of between 0.5 µg per µl.

30. Kit comprising the compound according to any of the claims 1 to 14.


SEQUENZPROTOKOLL

<110> Lion Bioscience AG
      VBC Genomics GmbH

<120> Branched Compound for Use in Nucleic Acid Detection and
      Analysis Reactions

<130> L-0009-00-EP-99

<140> EP
<141> 1999-12-21

<160> 4

<170> PatentIn Ver. 2.1

<210> 1
<211> 29
<212> DNA
<213> Künstliche Sequenz

<220>
<221> misc_structure
<222> (1)
<223> NH2-C6-

<220>
<223> Beschreibung der künstlichen Sequenz:
      künstliche

<400> 1
ttttttttta agcgcgcaat taaccctca                                   29

<210> 2
<211> 34
<212> DNA
<213> Künstliche Sequenz

<220>
<221> misc_structure
<222> (1)
<223> NH2-C6-

<220>
<223> Beschreibung der künstlichen Sequenz:Beschreibung
      der künstlichen Sequenz :künstliche

<400> 2
tttttttttt tttttaaaac gacggccagt gagc                                    34

<210> 3
<211> 15
<212> DNA
<213> Künstliche Sequenz

<220>
<221> misc_structure
<222> (1)
<223> (NH2-C6-ttt)2-branch-

<220>
<221> modified_base
<222> (15)
<223> NH2

<220>
<223> Beschreibung der künstlichen Sequenz: künstliche

<400> 3
tttttttttt ttttt                                                         15

<210> 4
<211> 15
<212> DNA
<213> Künstliche Sequenz

<220>
<221> misc_structure
<222> (1)
<223> (dt-COOH)2-branch-

<220>
<221> modified_base
<222> (15)
<223> NH2

<220>
<223> Beschreibung der künstlichen Sequenz: künstliche

<400> 4
tttttttttt ttttt                                                         15